# EUROPEAN PATENT SPECIFICATION

(11) **EP 3 195 811 B1**
(45) Date of publication and mention of the grant of the patent: **14.11.2018**
(21) Application number: 16168443.6
(22) Date of filing: 04.05.2016
(51) Int. Cl.: A61B 17/064, A61B 17/068, A61B 17/00

(54) **FIXING DEVICE FOR SOFT TISSUE**
BEFESTIGUNGSVORRICHTUNG FÜR WEICHES GEWEBE
DISPOSITIF DE FIXATION DE TISSU MOU

(30) Priority: 25.01.2016 CN 201610051820; 25.01.2016 CN 201620072770 U; 25.01.2016 CN 201610051764; 25.01.2016 CN 201620073762 U; 25.01.2016 CN 201610051818; 25.01.2016 CN 201610051765
(43) Date of publication of application: 26.07.2017
(73) Proprietor: TransEasy Medical Tech. Co., Ltd., 100027 Beijing (CN)
(72) Inventor: SUN, Jie, Beijing 100027 (CN); QIAO, Hongjiu, Beijing 100027 (CN); CHEN, Fan, Beijing 100027 (CN); DING, Lingcui, Beijing 100027 (CN); MENG, Kai, Beijing 100027 (CN)
(74) Representative: Rapisardi, Mariacristina

(56) References cited:
- WO-A1-2016/000246
- WO-A2-2004/112841
- US-A1- 2003 009 441
- US-A1- 2011 087 240
- US-A1- 2014 014 704

## Description

### FIELD OF THE INVENTION

The present invention relates to the field of medical equipment's technology, and more particularly to a fixing device for soft tissue with an absorbable screw or titanium screw.

### BACKGROUND OF THE INVENTION

Incisional hernia, which refers to a type of hernia that abdominal viscera or tissue protrudes from abdominal incision, is a common complication in laparotomy. The incision along a middle line of a lower portion of the abdomen has a high incidence of incisional hernia. The incisional hernia mostly occurs on longitudinal incision sites of the abdomen including incision dehiscence, incision infection and a secondary incision healing. A minority of the incisional hernia appears a long time after operation on the ones without incision dehiscence history. A morbidity of the incisional hernia is in the range of 2-11%, and an infected incision has an incisional hernia of up to about 40%. The incisional hernia on an abdominal wall is mainly treated by operation. Currently, the conventional repair method for the incisional hernia includes an open style hernia plastic operation with or without an artificial patch and a laparoscopic insional hernia repair operation. A recurrence rate of the incisional hernia after making a simple tissue suture repair operation is still up to 20-50%. According to a principle of incisional hernia classification in China, a medium-large or even huge scale of incisional hernia is more common in clinical trials. These types of incisional hernia are mainly performed with tension-free hernioplasty by the means of the artificial patch, which is capable of reducing effectively the recurrence rate of the incisional hernia to about 10%. Compared with open style hernia plastic operations, the laparoscopic insional hernia repair has characteristics as follows.
(1) The laparoscopic incisional hernia repair follows the principle of abdominal wall hernia surgery, and is in line with the theory of biological engineering mechanics of repairing for strengthening the rear wall, and the recurrence rate after the operation can reach 5%.
(2) The surgical approach is far away from the original incision hernia, so as to reduce the infection chance on implant sites of material, which is particularly suitable for the wound infection persons.
(3) A wide range of the abdominal tissue is not damaged to be free, and the original strength of the abdominal wall is retained as much as possible, which is more suitable for recurrent incisional hernia.
(4) Soft tissue capable of supplying blood is retained between skin on the hernia defect site and the artificial patch, and the soft tissue is capable of effectively cover and oppress the artificial patch, which is capable of facilitating the fibrous tissue to grow into an aperture of polypropylene mesh, so as to improve the tension strength between the artificial patch and the abdominal wall.
(5) Contents of free hernia in the pneumoperitoneum are more intuitively clear, and the force of gravity causes the contents of the hernia drooped naturally, so as to avoid the passive situation of intestinal injuries.
(6) An occult "pinhole hernia" is easier to find, so as to reduce the recurrence of abdominal hernia after operation caused thereby.
(7) Multi-point fixed patch is sutured and screwed in the abdominal cavity to reduce the uniformly dispersed tension.
(8) The area implanting the patch does has no skin incisions, so as to reduce the possibility of hematoma and liquid and replacement of a conventional drainage tube is not needed, so as to reduce the chance of contamination.
(9) The patch is fixed by non-abdominal wall penetration, so the postoperative pain is light, the duration of the operation is short and the patient recovers quickly.
(10) The laparoscopic operation is capable of not only reducing incisional infection, reducing postoperative recurrent rate but also achieving minimally invasive effects.
(11) The laparoscopic operation is technical demanding and requires a relative long learning time.

Based on the characteristics mentioned above, laparoscopic incisional hernia repair gradually attracts people's concern in recent years, and becomes more and more popular in the treatment of incisional hernia. The method for fixing patch in the laparoscopic hernia repair comprises completely suturing, completely nailing, combination of suturing, nailing and adhesive, and there is no uniform standard currently.

In the prior art, a speculum stapling device and a disposable screw case is disclosed in a patent application No. 200810009395.6, TYCO healthcare group. An absorbable screw nail for fixing hernia repair patch is disclosed in US 2007/0038220 A1, John Isbell Shipp, where the absorbable screw nail is made of a copolymer of lactide and glycolide. A surgical operation fixing method and device is disclosed in US 2007/0250064 A1, Davol, Inc., Cranston, RI. An I-shaped operation fixing device is disclosed in US 2003/0187465 A1, SOFRADIM PRODUCTION, Trevoux (FR). And a surgical spiral fixing device is disclosed in US5366479A, Origin Medsystems, Inc. Menlo park, Calif. The fixing device mentioned above solves the problems of time-consuming and inconvenient during the process of repairing suture, and furthermore, hemorrhage and trauma of the patient is decreased. The conventional surgical fixing device is in a type of a staple. Deformation fixed by the staple type fixing device needs other components such as an iron felt, and thus the structure is very complicated an the cost is high. A far-end of the one way screw nail has a penetrating point, and a near-end thereof has a T-shaped bar, wherein the T-shaped bar equally divides a diameter of the screw nail and provides a stress face. According to another embodiment, the screw nail is a double-sided screw, a far-end of the double-sided screw nail has two penetrating points and a near-end thereof has a connecting bar, wherein the connecting bar connects two spiral coils and equally divides diameters of the two spiral coils. In the prior art, the fixing device is generally a disposable product and may cause friction tissue injury on the human body during operation process.

US2003009441A1 discloses a surgical coil fastener applier for use in applying helical coil fasteners in surgical procedures, such as hernia repair, to affix surgical mesh to tissue. The coil fastener applier includes a housing having a handle extending therefrom and a trigger pivotally mounted on the housing. An elongated tubular portion extends from the housing and includes a drive rod rotatably supported therein and slidably supporting a plurality of coil fasteners thereon. A drive assembly is provided within the housing to rotate the drive rod and coil fasteners. The drive assembly includes an anti-reverse mechanism to allow rotation of the drive rod in only one direction. An actuation assembly is provided within the housing to convert the motion of the trigger into rotary motion for supply to the drive assembly. The drive assembly includes structure to limit the amount of rotary motion supplied to the drive assembly. The drive assembly further includes a ratchet and pawl mechanism to prevent a partial cycling of the coil fastener applier. Also provided are various coil fasteners for use with the coil fastener applier. The surgical coil fastener applier is configured and dimensioned to remove a coil fastener from tissue or drive a fastener previously applied to tissue further into the tissue. A lockout mechanism is provided to immobilize the drive rod relative to the elongated tubular portion.

WO2004112841A2 discloses an absorbable screw fastener and a method of firing with an applicator capable of applying a surgical fastener to tissue in order to form tissue connection to secure objects to tissue, the fastener including a body portion having a helical thread, a head portion disposed at the proximal end of the body portion. The head portion includes a driver-receiving configuration on its outer surface. The screw fastener further includes a cannulated center lumen with an opening extending from the head portion through the longitudinal length of the body portion.

WO2016000246A1 discloses an endoscopic surgical device is provided and includes a handle assembly, an elongated tube and a loading unit. The handle assembly includes a handle housing and a trigger operatively connected to the handle housing, and a drive mechanism which is actuated by the trigger. The elongated tube is selectively connectable to the handle assembly. The loading unit is selectively connectable to the elongated tube. The loading unit includes an outer tube, an inner tube and a plurality of surgical anchors. The outer tube defines a lumen therethrough and has a helical thread disposed within the lumen thereof. The inner tube is rotatably supported in the outer tube and operatively connectable to the drive mechanism, the inner tube defines a splined distal end, the splined distal end of the inner tube is defined by a pair of opposed longitudinally extending tines and a pair of opposed longitudinally extending channels; The plurality of surgical anchors are loaded in the splined distal end of the inner tube.

### SUMMARY OF THE PRESENT INVENTION

In order to solve the technical problems in the prior art, the present invention provides a fixing device for soft tissue with an absorbable screw or titanium screw as defined in the attached independent claim.

Further improvements and embodiments are provided in the dependent claims.

Accordingly, in order to achieve the objects mentioned above, the present invention adopts technical solutions as follows:
A fixing device for soft tissue includes: a handle, a tension spring, a trigger, a driving gear, a big helical gear, a small helical gear, a screw tube and a driving rod. A first end of the tension spring is mounted on a fixing post on an internal wall of the handle; a second end of the tension spring is arranged on an upper end of the trigger. One side of the upper end of the trigger is rotatablely connected to the internal wall of the handle via a rotation axis, a helical gear matching with the driving gear is arranged on the other side of the upper end of the trigger. The trigger extends outward from an inside of the handle and its lower end is located in the outside of the handle. The big helical gear and the driving gear are arranged concentrically, and the big helical gear is driven by the driving gear, outer teeth of the big helical gear match with outer teeth of the small helical gear. The driving rod is driven by a rotation axis of the small helical gear, the screw tube is arranged around the driving rod, a first end of the screw tube is arranged inside of the handle, and a second end of the screw tube is arranged outside of the handle, a catheter configured as a detachable replacement element that is removably connected on the second end of the screw pipe, and an absorbable screw or titanium screw is arranged inside of the catheter, a screw thread structure is arranged on a nailed beam of the absorbable screw, and a lead spring is provided on the screw thread structure.

In the present invention, a lubricant coating is covered on the catheter, which is beneficial to put the catheter having the fixing device for the soft tissue with the absorbable screw or titanium screw into human tissue smoothly, so as to prevent adhesion. In the prior art, the lubricant coating or an anti-adhesion coating may be for example silicon oil, such as dimethyl siloxane and the like. In the present invention, the lubricant coating containing Teflon is covered on the catheter, and a thickness of the lubricant coating is in the range of 3-5µm. A surface hardness of a lubricant compatible coating is in the range of Hv500-Hv1000, and a surface friction coefficient is in the range of 0.1-0.05. The lubricant coating further includes stable polymers selecting from a group consisting of one or more of the following: polymethyl methacrylate, polyacrylamide, polyacrylonitrile, polyether amide, poly-ethylenediamine, polycarbonate, polyketene, polyvinyl ether, polyvinyl ester, polyvinylpyrrolidone, polyethylene, polypropylene, polytetrafluoroethylene, polyolefin elastomer, polyisobutene, fluorosilicone, carboxymethyl chitosan, polyethylene terephthalate, poly-pentanoate, carboxymethyl cellulose, hydroxyethylcellulose, cellulose butyrate, cellulose acetate butyrate, ethyl-vinylacetate copolymer, chitosan and chitosan derivatives. Preferably, the lubricant coating further comprises antibacterial agent, the antibacterial agent can be a silver based antibacterial agent or a non-silver based antibacterial agent. For example, the silver based antibacterial agent, the silver based antibacterial agent can be silver particles, silver based antibacterial agent or non-silver based antibacterial agent based on glass carriers, or silver based antibacterial agent based on zeolite carrier. The antibacterial agent can be added to a combination by a conventional mixing method and dispersed in a lubricant compatible coating obtained by applying. The antibacterial agent is capable of preventing or inhibiting growth of microorganisms carried, thereby preventing infection caused by fixing operation.

Preferably, the absorbable screw is made of a degradable material. The degradable material is selected from a group consisting of at least one of the following: poly(L-lactide), poly(D, L-lactide), poly(L-lactide-co-D, L-lactide), poly(D,L-lactide-co-glycolide), poly-glycolide, poly(L-lactide-co-glycolide), poly(glycolide-co-trimethylene carbonate), poly(L-lactide-co-trimethylene carbonate), poly(D,L-lactide-co-trimethylene carbonate), poly(L-lactide-co-caprolactone), poly (amino acids), poly (ε-caprolactone), poly (δ-valerolactone), poly(γ-butyrolactone), poly (β-hydroxybutyrate), poly(DL-lactide), poly(L-lactide), ethylene carbonate (1,3-dioxolan-2-one), propylene carbonate (4-methyl-1,3-dioxolan-2-one), 1,3-propylene carbonate (1,3-dioxan-2-one) and tetramcthylene carbonate (1,3-dioxacyloheptane-2-one).

Preferably, the degradable material includes 10-15 wt% of poly(butylenes-co-ε-caprolactone carbonate) (PBCL) and 80-90wt% of poly(lactic-co-glycolic acid) (PLGA), the poly(lactic-co-glycolic acid) is synthesized by PDLA (foscolic acid) and PGA (poly glycolic acid), and a molar ratio of the PLDA to PGA is 40:60. Preferably, the degradable material further includes 1-5wt% of sulfo-N-hydroxy succinimide sodium.

The titanium screw is formed by helically buckling titanium alloy wire.

Preferably, the titanium alloy composes of 5.5-6.0wt% of Mo, 1.5-2.0wt% of Zr, 0.3-0.5wt% of Mg, 0.05-0.15wt% of Nb and a remaining amount of Ti. A micro-arc oxidation surface modification treatment for the titanium alloy wire is performed, and an electrolyte solution of the micro-arc oxidation surface modification treatment composes of 1.5-2.0wt% of sodium citrate, 0.10-0.20wt% of sodium borate, 0.05-0.10wt% of glycine and a remaining amount of water, and a voltage of the micro-arc oxidation surface modification treatment is in the range of 200-250V, a current density thereof is in the range of 0.02∼0.20 Adm-2, and a treatment time thereof is in the range of 10-20 min.

Compared with the prior art, the fixing device for soft tissue with the absorbable screw or titanium of the present invention has beneficial effects as follows.

The fixing device for soft tissue with the absorbable screw or titanium of the present invention is particularly suitable for laparoscopic hernia repair operations. Furthermore, the fixing device for soft tissue of the present invention has characteristics of anti-adhesion, convenient and reliable operation. In the present invention, the catheter is a detachable replacement element, so the fixing device can be reused after the catheter is replaced.

### BRIEF DESCRIPTION OF THE DRAWINGS

Fig. 1 is a structural schematic view of a fixing device for soft tissue according to a embodiment of the present invention.
Fig. 2 is an enlarged structure schematic view of an absorbable screw in a circle A in the Fig. 1.

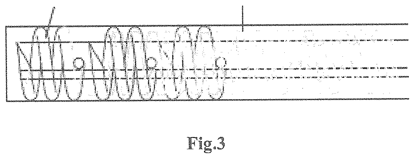

Fig. 3 is an enlarged structure schematic view of a titanium screw in the circle A in the Fig. 1.

### DETAILED DESCRIPTION OF THE EMBODIMENTS

Further description of a fixing device for soft tissue with an absorbable screw is illustrated with respect to embodiments of the present invention. These and other objectives, features, and advantages of the present invention will become apparent from the following detailed description, the accompanying drawings, and the appended claims.

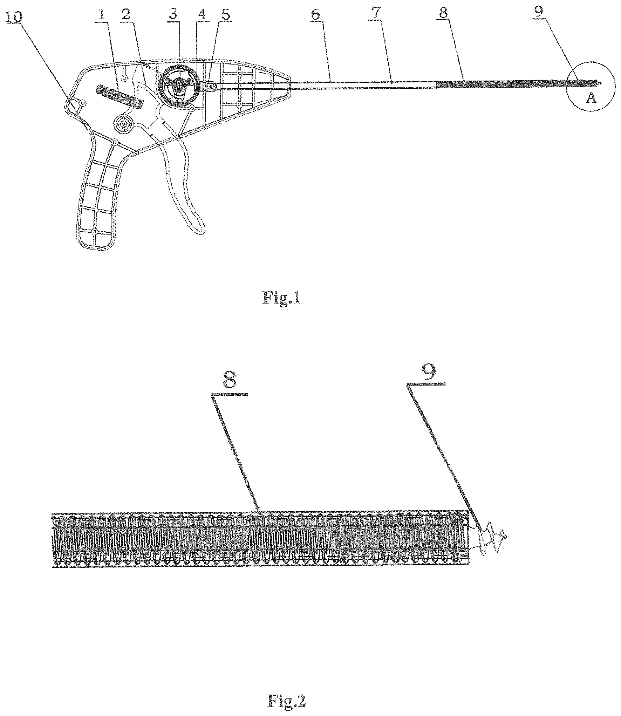

As shown in Figs. 1-2, according to a embodiment of the present invention, a fixing device for soft tissue with an absorbable screw includes a handle 10, an tension spring 1, a trigger 2, a driving gear 3, a big helical gear 4, a small helical gear 5, a screw tube 6 and a driving rod 7. A first end of the tension spring 1 is mounted on a fixing post on an internal wall of the handle 10, a second end of the tension spring 1 is arranged on an upper end of the trigger 2. One side of the upper end of the trigger 2 is rotatablely connected to the internal wall of the handle 10 via a rotation axis, a helical gear matching with the driving gear 3 is arranged on the other second side of the upper end of the trigger 2. The trigger 2 extends outward from an inside of the handle 10, and its lower end is located in the outside of the handle 10. The big helical gear 4 and the driving gear 3 are arranged concentrically, and the big helical gear 4 is driven by the driving gear 3, outer teeth of the big helical gear 4 match with outer teeth of the small helical gear 5. The driving rod 7 is driven by a rotation axis of the small helical gear 5, the screw tube 6 is arranged around the driving rod 7, a first end of the screw tube 6 is arranged inside of the handle 10, and a second end of the screw tube 6 is arranged ouitside of the handle 10, a catheter 8 is removably connected on the second end of the screw pipe, and an absorbable screw 9 is arranged inside of the catheter 8, a screw thread structure is arranged on a nailed beam of the absorbable screw 9, and a lead spring is provided on the screw thread structure. Specifically, a plurality of the absorbable screws are connected in series in the form of head to tail connection and put into the catheter with a spiral groove, where the heads of the absorbable screws toward an outlet of the catheter. In the embodiment, the trigger, the tension spring, the driving gear, the big helical gear and the small helical gear form a driving device of the fixing device for the soft tissue of the present invention. The driving rod is driven to rotate by the driving device in use under pulling the trigger, further the absorbable screw or titanium screw can be shot out of the catheter one by one when the laparoscopic hernia repair is performing.

In the present invention, a lubricant coating is covered on the catheter, which is beneficial to put the catheter having the fixing device for the soft tissue with the absorbable screw or titanium screw into human tissue smoothly, so as to prevent adhesion. In the prior art, the lubricant coating or an anti-adhesion coating may be for example silicon oil, such as dimethyl siloxane and the like. In the present invention, the lubricant coating containing Teflon is covered on the catheter, and a thickness of the lubricant coating is in the range of 3-5µm. A surface hardness of the lubricant coating is in the range of 3-5µm. A surface hardness of a lubricant compatible coating is in the range of Hv500-Hv1000, and a surface friction coefficient is in the range of 0.1-0.05. The lubricant coating further includes stable polymers selecting from a group consisting of one or more of the following: polymethyl methacrylate, polyacrylamide, polyacrylonitrile, polyether amide, poly-ethylenediamine, polycarbonate, polyketene, polyvinyl ether, polyvinyl ester, polyvinylpyrrolidone, polyethylene, polypropylene, polytetrafluoroethylene, polyolefin elastomer, polyisobutene, fluorosilicone, carboxymethyl chitosan, polyethylene terephthalate, poly-pentanoate, carboxymethyl cellulose, hydroxyethylcellulose, cellulose butyrate, cellulose acetate butyrate, ethyl-vinylacetate copolymer, chitosan and chitosan derivatives. Preferably, the lubricant coating contains 0.01-1.5wt% of antibacterial agent, the antibacterial agent can be silver based antibacterial agent or non-silver based antibacterial agent. For example, the silver based antibacterial agent, the silver based antibacterial agent can be silver particles, silver based antibacterial agent or non-silver based antibacterial agent based on glass carriers, or silver based antibacterial agent based on zeolite carrier. The antibacterial agent can be added to a combination by a conventional mixing method and dispersed in alubricant compatible coatingobtained by applying. The antibacterial agent is capable of preventing or inhibiting growth of microorganisms carried, thereby preventing infection caused by fixing operation.

### Embodiment 1

In the present invention, the absorbable screw is made of a degradable material. As an example, the degradable material is selected from a group consisting of at least one of the following: poly(L-lactide), poly(D, L-lactide), poly(L-lactide-co-D, L-lactide), poly(D,L-lactide-co-glycolide), poly-glycolide, poly(L-lactide-co-glycolide), poly(glycolide-co-trimethylene carbonate), poly(L-lactide-co-trimethylene carbonate), poly(D,L-lactide-co-trimethylene carbonate), poly(L-lactide-co-caprolactone), poly (amino acids), poly (ε-caprolactone), poly (δ-valerolactone), poly (γ-butyrolactone), poly (β-hydroxybutyrate), poly(DL-lactide), poly(L-lactide), ethylene carbonate (1,3-dioxolan-2-one), propylene carbonate (4-methyl-1,3-dioxolan-2-one), 1,3-propylene carbonate (1,3-dioxan-2-one) and tetramcthylene carbonate (1,3-dioxacyloheptane-2-one).

As an improvement, the degradable material includes 10-15 wt% of poly(butylenes-co-ε-caprolactone carbonate) (PBCL) and 80-90wt% of poly(lactic-co-glycolic acid) (PLGA), the poly(lactic-co-glycolic acid) is synthesized by PDLA (foscolic acid) and PGA (poly glycolic acid), where a molar ratio of the PDLA to the PGA is 40:60. Preferably, the degradable material further includes 1-5wt% of sulfo-N-hydroxy succinimide sodium. The static degradation testing based on Hanks' balanced salt solution indicates that: adding PBCL is capable of stabilizing a time which makes the have a weight loss of 90% within 80-90 days. Thus the degradable materials have excellent degradation performance, small stimulation and excellent compatibility. In addition, adding sulfo-N-hydroxy succinimide sodium is capable of facilitating hernia coalescence.

In the present invention, the absorbable screw is obtained by processes of raw material mixing, melt extruding and machining. As an example, raw material combination of the degradable materials is sent into a blender mixer to be evenly mixed, where a mixing temperature is controlled below 80oC. Mixed materials are cooled and then melt extruded by a single-screw extruder. Temperature on each section of the single-screw extruder is in the range of 150∼210oC. Melt blending materials obtained are processed into a round bar after being extruded. A mechanic performance testing shows that a tensile strength of the round bar is capable of reaching 50-60MPa, and a bending strength thereof is capable of reaching 100-120MPa. The stretched round bar is cut into sections by certain length, and then is performed with lathe processing according to screw thread parameters and screw structures, the absorbable screw is obtained. In the present invention, the absorbable screw adopts a shape of the conventional screw in the prior art, e.g., a front half of the whole body is a cone shape, and a back half thereof is a post shape, or the whole body is a circular truncated cone, such that the absorbable screw can be positioned accurately by a guide pin in the operation. Parameters of the screw thread are not limited, and a deep and flattened thread is preferred.

### Embodiment 2

In the present invention, the titanium screw is formed by helically buckling titanium alloy wire. The titanium alloy composes of 5.5-6.0wt% of Mo, 1.5-2.0wt% of Zr, 0.3-0.5wt% of Mg, 0.05-0.15wt% of Nb and a remaining amount of Ti. In the present invention, by solution treatment, extension strength of the titanium alloy can reach 820-900MPa, yield strength can reach 800-850MPa, an elongation ratio can reach 25-35% and a fracture toughness can reach 85-90 MPam^{-1/2}, and thus malleability and toughness of the titanium alloy is good. During machining process, a micro-arc oxidation surface modification treatment for the titanium alloy wire is performed, and an electrolyte solution of the micro-arc oxidation surface modification treatment composes of 1.5-2.0wt% of sodium citrate, 0.10-0.20wt% of sodium borate, 0.05-0.10wt% of glycine and a remaining amount of water. After the modification treatment, in the alloy wire, which is added with an appropriate amount of Mg and Zr and in a Hank's solution, its anodizing curve can maintain in a stable passive state at a voltage of 0.5-2.5V, and a dissolution quantity of metallic ion is small (100ppmcm⁻² below), thereby significantly improving corrosion resistance and biocompatibility of the titanium screw. Specifically, a voltage of the micro-arc oxidation treatment is in the range of 200-250V and a current density thereof is in the range of 0.02-0.20 Adm⁻², and treatment time is in the range of 10-20 min.

## Claims

1. A fixing device for soft tissue, comprising: a handle (10), an tension spring (1), a trigger (2), a driving gear (3), a big helical gear (4), a small helical gear (5), a screw tube (6) and a driving rod (7), **characterized in that**, a first end of the tension spring (1) is mounted on a fixing post on an internal wall of the handle (10), a second end of the tension spring (1) is arranged on an upper end of the trigger (2), one side of the upper end of the trigger (2) is rotatablely connected to the internal wall of the handle (10) via a rotation axis; helical teeth matching with the driving gear (3) is arranged on the other side of the upper end of the trigger (2), the trigger (2) extends outward from an inside of the handle (10) and its lower end located in the outside of the handle (10), the big helical gear (4) and the driving gear (3) are arranged concentrically, and the big helical gear (4) is driven by the driving gear (3), outer teeth of the big helical gear (4) match with outer teeth of the small helical gear (5); the driving rod (7) is driven by a rotation axis of the small helical gear (5), the screw tube (6) is arranged around the driving rod (7), a first end of the screw tube (6) is arranged inside of the handle (10), and a second end of the screw tube (6) is arranged outside of the handle (10), a catheter (8) configured as a detachable replacement element that is removably connected on the second end of the screw tube (6), and an absorbable screw (9) or titanium screw is arranged inside of the catheter (8), a screw thread structure is formed on a nailed beam of only the absorbable screw (9), and a lead spring is arranged on the screw thread structure.

2. The fixing device for soft tissue of claim 1, wherein a lubricant coating containing Teflon is covered on the catheter, and a thickness of the lubricant coating is in the range of 3-5µm.

3. The fixing device for soft tissue of claim 2, wherein a surface hardness of the lubricant coating is in the range of Hv500-Hv1000, and a its surface friction coefficient is in the range of 0.1-0.05.

4. The fixing device for soft tissue of claim 2, wherein the lubricant coating further comprises stable polymers selecting from a group consisting of one or more of the following: polymethyl methacrylate, polyacrylamide, polyacrylonitrile, polyether amide, poly-ethylenediamine, polycarbonate, polyketene, polyvinyl ether, polyvinyl ester, polyvinylpyrrolidone, polyethylene, polypropylene, polytetrafluoroethylene, polyolefin elastomer, polyisobutene, fluorosilicone, carboxymethyl chitosan, polyethylene terephthalate, poly-pentanoate, carboxymethyl cellulose, hydroxyethylcellulose, cellulose butyrate, cellulose acetate butyrate, ethyl-vinylacetate copolymer, chitosan and chitosan derivatives.

5. The fixing device for soft tissue of claim 2, wherein the lubricant coating further comprises antibacterial agent.

6. The fixing device for soft tissue of claim 1, wherein the absorbable screw (9) is made of degradable material.

7. The fixing device for soft tissue of claim 6, wherein the degradable material is selected from a group consisting of at least one of the following: poly(L-lactide), poly(D, L-lactide), poly(L-lactide-co-D, L-lactide), poly(D,L-lactide-co-glycolide), poly-glycolide, poly(L-lactide-co-glycolide), poly(glycolide-co-trimethylene carbonate), poly(L-lactide-co-trimethylene carbonate), poly(D,L-lactide-co-trimethylene carbonate), poly(L-lactide-co-caprolactone), poly (amino acids), poly (ε-caprolactone), poly (δ -valerolactone), poly (γ-butyrolactone), poly (β-hydroxybutyrate), poly(DL-lactide), poly(L-lactide), ethylene carbonate (1,3-dioxolan-2-one), propylene carbonate (4-methyl-1,3-dioxolan-2-one), 1,3-propylene carbonate (1,3-dioxan-2-one) and tetramcthylene carbonate (1,3-dioxacyloheptane-2-one).

8. The fixing device for soft tissue of claim 6, wherein the degradable material comprises 10-15 wt% of poly(butylenes-co-ε-caprolactone carbonate) (PBCL) and 80-90wt% of poly(lactic-co-glycolic acid) (PLGA), the poly(lactic-co-glycolic acid) is synthesized by PDLA (foscolic acid) and PGA (poly glycolic acid) and a molar ratio of the PLDA to PGA is 40:60.

9. The fixing device for soft tissue of claim 8, wherein the degradable material further comprises 1-5wt% of sulfo-N-hydroxy succinimide sodium.

10. The fixing device for soft tissue of claim 1, wherein the titanium screw is formed by helically buckling a titanium alloy wire.

11. The fixing device for soft tissue of claim 10, wherein the titanium alloy composes of 5.5-6.0wt% of Mo, 1.5-2.0wt% of Zr, 0.3-0.5wt% of Mg, 0.05-0.15wt% of Nb and a remaining amount of Ti.

12. The fixing device for soft tissue of claim 10, wherein a micro-arc oxidation surface modification treatment for the titanium alloy wire is performed, and an electrolyte of the micro-arc oxidation surface modification treatment, and an electrolyte solution of the micro-arc oxidation surface modification treatment composes of 1.5-2.0wt% of sodium citrate, 0.10-0.20wt% of sodium borate, 0.05-0.10wt% of glycine and a remaining amount of water, wherein a voltage of the micro-arc oxidation surface modification treatment is in the range of 200-250V and its current density is in the range of 0.02-0.20 Adm-2, and its treatment time is in the range of 10-20 min.

## Patentansprüche

1. Befestigungsvorrichtung für weiches Gewebe, umfassend: einen Griff (10), eine Zugfeder (1), einen Abzug (2), ein Antriebsrad (3), ein großes Schrägstirnrad (4), ein kleines Schrägstirnrad (5), ein Schraubenrohr (6) und eine Antriebsstange (7), **dadurch gekennzeichnet, dass** ein erstes Ende der Zugfeder (1) an einem Befestigungspfosten an einer Innenwand des Griffs (10) montiert ist, ein zweites Ende der Zugfeder (1) an einem oberen Ende des Abzuges (2) angeordnet ist, eine Seite des oberen Endes des Abzuges (2) über eine Drehachse drehbar mit der Innenwand des Griffes (10) verbunden ist; wobei die Schrägverzahnung, die mit dem Antriebsrad (3) übereinstimmt, auf der anderen Seite des oberen Endes des Abzuges (2) angeordnet ist, der Abzug (2) sich von einer Innenseite des Griffes (10) nach außen erstreckt und sein unteres Ende sich in der Außenseite des Griffes (10) befindet, das große Schrägstirnrad (4) und das Antriebsrad (3) konzentrisch angeordnet sind und das große Schrägstirnrad (4) durch das Antriebsrad (3) angetrieben wird, wobei die Außenverzahnung des großen Schrägstirnrades (4) mit der Außenverzahnung des kleinen Schrägstirnrades (5) übereinstimmt; die Antriebsstange (7) von einer Drehachse des kleinen Schrägstirnrades (5) angetrieben wird, das Schraubenrohr (6) rund um die Antriebsstange (7) angeordnet ist, ein erstes Ende des Schraubenrohres (6) innerhalb des Griffs (10) angeordnet ist und ein zweites Ende des Schraubenrohres (6) außerhalb des Griffes (10) angeordnet ist, einen Katheter (8), der als abnehmbares Ersatzelement ausgelegt ist, das am zweiten Ende des Schraubenrohres (6) lösbar verbunden ist, und eine resorbierbare Schraube (9) oder Titanschraube innerhalb des Katheters (8) angeordnet ist, eine Schraubengewinde-Struktur an einem genagelten Träger nur der resorbierbaren Schraube (9) gebildet ist und eine Leitfeder an der Schraubengewinde-Struktur angeordnet ist.

2. Befestigungsvorrichtung für weiches Gewebe nach Anspruch 1, wobei eine Teflon enthaltende Gleitbeschichtung auf dem Katheter überzogen ist und eine Dicke der Gleitbeschichtung im Bereich von 3-5 µm liegt.

3. Befestigungsvorrichtung für weiches Gewebe nach Anspruch 2, wobei eine Oberflächenhärte der Gleitbeschichtung im Bereich von Hv500-Hv1000 und ein Oberflächenreibungskoeffizient im Bereich von 0,1-0,05 liegt.

4. Befestigungsvorrichtung für weiches Gewebe nach Anspruch 2, wobei die Gleitbeschichtung ferner stabile Polymere umfasst, die aus einer Gruppe ausgewählt sind, die aus einem oder mehreren der folgenden besteht: Polymethylmethacrylat, Polyacrylamid, Polyacrylnitril, Polyetheramid, Polyethylendiamin, Polycarbonat, Polyketen, Polyvinylether, Polyvinylester, Polyvinylpyrrolidon, Polyethylen, Polypropylen, Polytetrafluorethylen, Polyolefinelastomer, Polyisobuten, Fluorsilikon, Carboxymethylchitosan, Polyethylenterephthalat, Poly-pentanoat, Carboxymethylcellulose, Hydroxyethylcellulose, Cellulosebutyrat, Celluloseacetatbutyrat, Ethyl-Vinylacetat-Copolymer, Chitosan und Chitosanderivate.

5. Befestigungsvorrichtung für weiches Gewebe nach Anspruch 2, wobei die Gleitbeschichtung ferner ein antibakterielles Mittel enthält.

6. Befestigungsvorrichtung für weiches Gewebe nach Anspruch 1, wobei die resorbierbare Schraube (9) aus abbaubarem Material besteht.

7. Befestigungsvorrichtung für weiches Gewebe nach Anspruch 6, wobei das abbaubare Material aus einer Gruppe ausgewählt ist, die aus mindestens einem der folgenden besteht: Poly(L-Lactid), Poly(D, L-Lactid), Poly(L-Lactid-co-D, L-Lactid), Poly(D,L-Lactid-co-glycolid), Poly-glycolid, Poly(L-Lactid-co-glycolid), Poly(glycolid-co-trimethylencarbonat), Poly(L-Lactid-co-trimethylencarbonat), Poly(D,L-Lactid-co-trimethylencarbonat), Poly(L-Lactid-co-caprolacton), Poly(Aminosäuren), Poly (ε-Caprolacton), Poly (δ-Valerolacton), Poly (γ-Butyrolacton), Poly (β-Hydroxybutyrat), Poly(DL-Lactid), Poly(L-Lactid), Ethylencarbonat (1,3-Dioxolan-2-on), Propylencarbonat (4-Methyl-1,3-Dioxolan-2-on), 1,3-Propylencarbonat (1,3-Dioxan-2-on) und Tetramcthylencarbonat (1,3-Dioxacyloheptan-2-on).

8. Befestigungsvorrichtung für weiches Gewebe nach Anspruch 6, wobei das abbaubare Material 10-15 Gew.-% Poly(butylene-co-ε-caprolactoncarbonat) (PBCL) und 80-90 Gew.-% Poly(milch-co-glykolsäure) (PLGA) umfasst, die Poly(milch-co-glykolsäure) wird durch PDLA (Foskolsäure) und PGA (Polyglykolsäure) synthetisiert und ein Molverhältnis von PLDA zu PGA beträgt 40:60.

9. Befestigungsvorrichtung für weiches Gewebe nach Anspruch 8, wobei das abbaubare Material ferner 1-5 Gew.-% Sulfo-N-Hydroxy-Succinimid-Natrium enthält.

10. Befestigungsvorrichtung für weiches Gewebe nach Anspruch 1, wobei die Titanschraube durch schraubenförmiges Knicken eines Titanlegierungsdrahtes gebildet wird.

11. Befestigungsvorrichtung für weiches Gewebe nach Anspruch 10, wobei die Titanlegierung aus 5,5-6,0 Gew.-% Mo, 1,5-2,0 Gew.-% Zr, 0,3-0,5 Gew.-% Mg, 0,05-0,15 Gew.-% Nb und einer Restmenge an Ti besteht.

12. Befestigungsvorrichtung für weiches Gewebe nach Anspruch 10, wobei eine Mikrolichtbogen-Oxidationsoberflächenmodifikationsbehandlung für den Titanlegierungsdraht durchgeführt wird, und ein Elektrolyt der Mikrolichtbogen-Oxidationsoberflächenmodifikationsbehandlung und eine Elektrolytlösung der Mikrolichtbogen-Oxidationsoberflächenmodifikationsbehandlung aus 1.5-2.0 Gew.-% Natriumcitrat, 0,10-0,20 Gew.-% Natriumborat, 0,05-0,10 Gew.-% Glycin und einer verbleibenden Menge an Wasser besteht, wobei eine Spannung der Mikrolichtbogen-Oxidationsoberflächenmodifikationsbehandlung im Bereich von 200-250V liegt und ihre Stromdichte im Bereich von 0,02∼0,20 Adm-2 liegt und ihre Behandlungszeit im Bereich von 10-20 min liegt.

## Revendications

1. Dispositif de fixation de tissu mou, comprenant : une poignée (10), un ressort de tension (1), une gâchette (2), un engrenage menant (3), un grand engrenage hélicoïdal (4), un petit engrenage hélicoïdal (5), un tube fileté (6) et une tige d'entraînement (7), **caractérisé en ce qu'**une première extrémité du ressort de tension (1) est montée sur un tenon de fixation sur une cloison intérieure de la poignée (10), une seconde extrémité du ressort de tension (1) est disposée sur une extrémité supérieure de la gâchette (2), un côté de l'extrémité supérieure de la gâchette (2) est relié de façon rotative à la cloison intérieure de la poignée (10) via un axe de rotation ; des dents hélicoïdales correspondant à l'engrenage menant (3) sont disposées sur l'autre côté de l'extrémité supérieure de la gâchette (2), la gâchette (2) se prolongeant vers l'extérieur à partir d'un intérieur de la poignée (10) et de son extrémité inférieure située à l'extérieure de la poignée (10), le grand engrenage hélicoïdal (4) et l'engrenage menant (3) sont disposés concentriquement, et le grand engrenage hélicoïdal (4) est entraîné par l'engrenage menant (3), les dents extérieures du grand engrenage hélicoïdal (4) correspondant aux dents extérieures du petit engrenage hélicoïdal (5) ; la tige d'entraînement (7) est actionnée par un axe de rotation du petit engrenage hélicoïdal (5), le tube fileté (6) est disposé autour de la tige d'entraînement (7), une première extrémité du tube fileté (6) est disposée à l'intérieur de la poignée (10) et une seconde extrémité du tube fileté (6) est disposée à l'extérieur de la poignée (10), un cathéter (8) configuré comme étant un élément de remplacement détachable étant relié de façon amovible à la seconde extrémité du tube fileté (6), et une vis résorbable (9) ou une vis en titane est disposée à l'intérieur du cathéter (8), une structure à filetage est formée sur un profilé cloué que de la vis résorbable (9), et un ressort fileté est disposé sur la structure à filetage.

2. Dispositif de fixation de tissu mou selon la revendication 1, dans lequel un revêtement lubrifiant contenant du téflon recouvre le cathéter et une épaisseur du revêtement lubrifiant est comprise entre 3-5 µm.

3. Dispositif de fixation de tissu mou selon la revendication 2, dans lequel le degré de dureté de la surface du revêtement lubrifiant est comprise entre 500 Hv et 1 000 Hv et son coefficient de frottement superficiel est compris entre 0,1-0,05.

4. Dispositif de fixation de tissu mou selon la revendication 2, dans lequel le revêtement lubrifiant comprend de plus des polymères stables choisis dans un groupe consistant en un ou plusieurs des éléments suivants : polyméthacrylate de méthyle, polyacrylamide, polyacrylonitrile, amide de polyéther, poly-éthylènediamine, polycarbonate, polycétone, polyvinyle-éther, ester de polyvinyle, polyvinylpyrrolidone, polyéthylène, polypropylène, polytétrafluoroéthylène, élastomère de polyoléfine, polyisobutène, fluorosilicone, chitosane de carboxyméthyle, polyéthylène téréphtalate, poly-pentanoate, carboxyméthylcellulose, glycocellulose, butyrate de cellulose, acétobutyrate de cellulose, copolymère d'éthylène et d'acétate de vinyle, chitosane et dérivés de chitosane.

5. Dispositif de fixation de tissu mou selon la revendication 2, dans lequel le revêtement lubrifiant comprend de plus un agent antibactérien.

6. Dispositif de fixation de tissu mou selon la revendication 1, dans lequel la vis résorbable (9) est constituée d'un matériau dégradable.

7. Dispositif de fixation de tissu mou selon la revendication 6, dans lequel le matériau dégradable est choisi à partir d'un groupe consistant en au moins un des éléments suivants : poly(L-lactide), poly(D, L-lactide), poly(L-lactide-co-D, L-lactide), poly(D,L-lactide-co-glycolide), poly-glycolide, poly(L-lactide-co-glycolide), poly(glycolide-co-carbonate de triméthylène), poly(L-lactide-co-carbonate de triméthylène), poly(D,L-lactide-co-carbonate de triméthylène), poly(L-lactide-co-caprolactone), poly (acides aminés), poly (ε-caprolactone), poly (δ-valérolactone), poly (γ-butyrolactone), poly (β-hydroxybutyrate), poly(DL-lactide), poly(L-lactide), carbonate d'éthylène (1,3-dioxolan-2-one), de carbonate propylène (4-méthyl-1,3-dioxolan-2-one), 1,3-carbonate de propylène (1,3-dioxan-2-one) et carbonate de tétraméthylène (1,3-dioxacyloheptane-2-one).

8. Dispositif de fixation de tissu mou selon la revendication 6, dans lequel le matériau dégradable comprend 10-15 % en poids de poly(butylènes-co-ε-carbonate de caprolactone) (PBCL) et 80-90 % en poids de poly(acide lactique-co-glycolique) (PLGA), le poly(acide lactique-co-glycolique) est synthétisé par PDLA (acide foscolique) et PGA (acide polyglycolique) et un rapport molaire de PLDA par rapport à PGA correspondant à 40:60.

9. Dispositif de fixation de tissu mou selon la revendication 8, dans lequel le matériau dégradable comprend de plus 1-5 % en poids de sulfo-N-hydroxy succinimide sodium.

10. Dispositif de fixation de tissu mou selon la revendication 1, dans lequel la vis en titane est formée par gauchissement de façon hélicoïdale d'un fil en alliage de titane.

11. Dispositif de fixation de tissu mou selon la revendication 10, dans lequel l'alliage de titane se compose de 5,5-6,0 % en poids de Mo, 1,5-2,0 % en poids de Zr, 0,3-0,5 % en poids de Mg, 0,05-0,15 % en poids de Nb et d'une quantité restante de Ti.

12. Dispositif de fixation de tissu mou selon la revendication 10, dans lequel est réalisé un traitement de modification de surface par oxydation micro-arc pour le fil en alliage de titane, et un électrolyte du traitement de modification de surface par oxydation micro-arc, et une solution électrolyte du traitement de modification de surface par oxydation micro-arc se compose de 1,5-2,0 % en poids de citrate de sodium, 0,10-0,20 % en poids de borate de sodium, 0,05-0,10 % en poids de glycine et une quantité restante d'eau, dans lequel une tension du traitement de modification de surface par oxydation micro-arc est comprise entre 200-250 V et sa densité de courant est comprise entre 0,02∼0,20 Adm⁻² et sa durée de traitement est comprise entre 10-20 min.
